# EUROPEAN PATENT SPECIFICATION

(11) **EP 3 576 845 B1**
(45) Date of publication and mention of the grant of the patent: **07.06.2023**
(21) Application number: 18704767.5
(22) Date of filing: 30.01.2018
(51) Int. Cl.: A61Q 1/04, A61K 8/58, A61K 8/92, A61K 8/89

(54) **TOPCOAT FOR PERMANENT LIP MAKE UP**
TOPCOAT FÜR PERMANENTES LIPPEN MAKEUP
TOP COAT POUR LE MAQUILLAGE PERMANENT DES LÈVRES

(30) Priority: 31.01.2017 US 201715420888
(43) Date of publication of application: 11.12.2019
(73) Proprietor: L'OREAL, 75008 Paris (FR); Zhu, Chao, Clark, NJ 07066 (US); Rosario-Melendez, Roselin, Clark, NJ 07066 (US); Desteno, Susan, Ashley, Clark, NJ 07066 (US); El-Khouri, Rita, Jaky, Clark, NJ 07066 (US)
(72) Inventor: ZHU, Chao, Clark, NJ 07066 (US); ROSARIO-MELENDEZ, Roselin, Clark, NJ 07066 (US); DESTENO, Susan, Ashley, Clark, NJ 07066 (US); EL-KHOURI, Rita, Jaky, Clark, NJ 07066 (US)
(74) Representative: Cabinet Nony
(86) International application number: PCT/US2018/015960
(87) International publication number: WO 2018/144460

(56) References cited:
- EP-A1- 1 938 864
- WO-A1-2008/079478
- WO-A1-2009/156375
- US-A1- 2009 092 567

## Description

### CROSS-REFERENCE TO RELATED APPLICATIONS

This application claims the benefit of priority from U.S. non-provisional application no. 15/420,888, filed January 31, 2017.

### FIELD OF THE INVENTION

The present invention is as defined in the appended claims and relates to a topcoat composition for long-wear lip compositions comprising at least one silicone film-forming agent, as well as to systems, kits and methods of treating, making-up and enhancing the appearance of lips, including a long-wear lip composition comprising at least one silicone film-forming agent and a topcoat composition for application to the long-wear lip composition. The topcoat composition does not materially affect the transfer-resistance of the lip composition.

### DISCUSSION OF THE BACKGROUND

Many cosmetic compositions, including pigmented cosmetics such as lipsticks or lip colors, have been formulated in an attempt to possess long-wearing properties upon application. Unfortunately, many of these compositions do not generally possess both good long-wear/transfer-resistance properties as well as good application properties, good comfort properties and/or good appearance properties (for example, shine properties).

For example, with respect to lip compositions, commercial products containing silicon resins such as MQ resins are known. Such products are known to provide good long wear properties/transfer-resistance. However, such products possess poor application properties and/or poor feel upon application (for example, feels rough). See e.g. EP 1 938 864 A1 in this context.

Typically, a second composition (topcoat) is separately applied to such products to improve poor properties of the compositions to make the products acceptable to consumers. However, topcoat compositions tend to decrease the long-wear/transfer-resistance properties of the lip compositions, thereby rendering the long-wear/transfer resistant composition less acceptable to consumers and less acceptable for their intended purpose.

Thus, there remains a need for improved lip compositions and systems having improved cosmetic properties, particularly good transfer-resistance, feel and shine characteristics upon application.

Accordingly, one aspect of the present invention is a topcoat composition for long-wear lip compositions which provide or maintain good cosmetic properties such as, for example, good transfer-resistance, feel and shine properties upon application to a long-wear lip composition.

### SUMMARY OF THE INVENTION

The present invention is defined in the appended claims and relates to a topcoat composition for a long-wear lip composition comprising at least one silicone film-forming agent, wherein the topcoat composition comprises at least one phenylated silicone oil and at least one wax. Preferably, the topcoat composition consists essentially of at least one phenylated silicone oil and at least one wax. The topcoat composition does not comprise diglyceryl polyacyladipate. Preferably, when the topcoat composition is applied to the long-wear lip composition, the topcoat composition does not inhibit the transfer-resistance of the long-wear lip composition.

The present invention relates to kits comprising (1) a long-wear lip composition comprising at least one silicone film-forming agent, and (2) a topcoat composition comprising at least one phenylated silicone oil and at least one wax. Preferably, the topcoat composition consists essentially of at least one phenylated silicone oil and at least one wax. Preferably, the topcoat composition does not comprise diglyceryl polyacyladipate. Preferably, when the topcoat composition is applied to the long-wear lip composition, the topcoat composition does not inhibit the transfer-resistance of the long-wear lip composition.

The present invention relates to cosmetic systems comprising (1) a long-wear lip composition comprising at least one silicone film-forming agent, and (2) a topcoat composition comprising at least one phenylated silicone oil and at least one wax. Preferably, the topcoat composition consists essentially of at least one phenylated silicone oil and at least one wax. Preferably, the topcoat composition does not comprise diglyceryl polyacyladipate. Preferably, when the topcoat composition is applied to the long-wear lip composition, the topcoat composition does not inhibit the transfer-resistance of the long-wear lip composition.

The present invention relates to methods for enhancing the appearance of lips comprising applying a long-wear lip composition comprising at least one silicone film-forming agent to the lips, and (2) applying a topcoat composition comprising at least one phenylated silicone oil and at least one wax to the long-wear lip composition. Preferably, the topcoat composition consists essentially of at least one phenylated silicone oil and at least one wax. Preferably, the topcoat composition does not comprise diglyceryl polyacyladipate. Preferably, when the topcoat composition is applied to the long-wear lip composition, the topcoat composition does not inhibit the transfer-resistance of the long-wear lip composition.

The present invention relates to methods for making-up lips comprising applying a long-wear lip composition comprising at least one silicone film-forming agent to the lips, and (2) applying a topcoat composition comprising at least one phenylated silicone oil and at least one wax to the long-wear lip composition. Preferably, the topcoat composition consists essentially of at least one phenylated silicone oil and at least one wax. Preferably, the topcoat composition does not comprise diglyceryl polyacyladipate. Preferably, when the topcoat composition is applied to the long-wear lip composition, the topcoat composition does not inhibit the transfer-resistance of the long-wear lip composition.

The present invention relates to methods for treating lips comprising applying a long-wear lip composition comprising at least one silicone film-forming agent to the lips, and (2) applying a topcoat composition comprising at least one phenylated silicone oil and at least one wax to the long-wear lip composition. Preferably, the topcoat composition consists essentially of at least one phenylated silicone oil and at least one wax. Preferably, the topcoat composition does not comprise diglyceryl polyacyladipate. Preferably, when the topcoat composition is applied to the long-wear lip composition, the topcoat composition does not inhibit the transfer-resistance of the long-wear lip composition.

It is to be understood that both the foregoing general description and the following detailed description are exemplary and explanatory only, and are not restrictive of the invention.

### DETAILED DESCRIPTION OF THE INVENTION

According to the invention, the term "anhydrous" refers to a composition not containing any water, that is to say a composition in which the water that may be present comes only from the water of crystallization or of adsorption of the starting materials. In any case, an anhydrous composition contains less than 5% by weight of water, preferably less than 1% by weight, and better still less than 0.5% by weight of water, relative to the total weight of the composition.

As used herein, the expression "at least one" means one or more and thus includes individual components as well as mixtures/combinations.

Other than in the operating examples, or where otherwise indicated, all numbers expressing quantities of ingredients and/or reaction conditions are to be understood as being modified in all instances by the term "about," meaning within 10% to 15% of the indicated number (e.g. "about 10%" means 9% - 11% and "about 2%" means 1.8% - 2.2%).

"Film former" or "film forming agent" or "film forming polymer" or "film forming resin" as used herein mean a polymer or resin that leaves a film on the substrate to which it is applied, for example, after a solvent accompanying the film former has evaporated, absorbed into and/or dissipated on the substrate.

"Transfer resistance" as used herein refers to the quality exhibited by compositions that are not readily removed by contact with another material, such as, for example, a glass, an item of clothing or the skin, for example, when eating or drinking. Transfer resistance may be evaluated by any method known in the art for evaluating such. For example, transfer resistance of a composition may be evaluated by a "kiss" test. The "kiss" test may involve application of the composition to human keratin material such as hair, skin or lips followed by rubbing a material, for example, a sheet of paper, against the hair, skin or lips after expiration of a certain amount of time following application, such as 2 minutes after application. Similarly, transfer resistance of a composition may be evaluated by the amount of product transferred from a wearer to any other substrate, such as transfer from the hair, skin or lips of an individual to a collar when putting on clothing after the expiration of a certain amount of time following application of the composition to the hair, skin or lips. The amount of composition transferred to the substrate (e.g., collar, or paper) may then be evaluated and compared. For example, a composition may be transfer resistant if a majority of the product is left on the wearer's hair, skin or lips. Further, the amount transferred may be compared with that transferred by other compositions, such as commercially available compositions. In a preferred embodiment of the present invention, little or no composition is transferred to the substrate from the hair, skin or lips.

"Long wear" compositions as used herein, refers to compositions where color remains the same or substantially the same as at the time of application, as viewed by the naked eye, after an extended period of time. Long wear properties may be evaluated by any method known in the art for evaluating such properties. For example, long wear may be evaluated by a test involving the application of a composition to human hair, skin or lips and evaluating the color of the composition after an extended period of time. For example, the color of a composition may be evaluated immediately following application to hair, skin or lips and these characteristics may then be re-evaluated and compared after a certain amount of time. Further, these characteristics may be evaluated with respect to other compositions, such as commercially available compositions. For lip compositions, "long wear" typically means the composition remains on the lips at least about 4 hours up to about 24 hours, and retains rich color even after eating.

"Liquid" or "liquid cosmetic" or "liquid lipstick" or "liquid composition" means a composition having a fixed volume, flows to cover the bottom and assumes the shape of the portion of the container it fills and is slightly compressible (as disclosed in General chemistry, Fourth Edition 2005, p.434

"Tackiness" as used herein refers to the adhesion between two substances. For example, the more tackiness there is between two substances, the more adhesion there is between the substances. To quantify "tackiness," it is useful to determine the "work of adhesion" as defined by IUPAC associated with the two substances. Generally speaking, the work of adhesion measures the amount of work necessary to separate two substances. Thus, the greater the work of adhesion associated with two substances, the greater the adhesion there is between the substances, meaning the greater the tackiness is between the two substances.

Work of adhesion and, thus, tackiness, can be quantified using acceptable techniques and methods generally used to measure adhesion and the one far along described.

"Substituted" as used herein, means comprising at least one substituent. Non-limiting examples of substituents include atoms, such as oxygen atoms and nitrogen atoms, as well as functional groups, such as amine groups, ether groups, alkoxy groups, acyloxyalky groups, oxyalkylene groups, polyoxyalkylene groups, carboxylic acid groups, amine groups, acylamino groups, amide groups, halogen containing groups, ester groups, thiol groups, sulphonate groups, thiosulphategroups, siloxane groups, and polysiloxane groups. The substituent(s) may be further substituted.

"Comprising" it is meant that other steps and/or ingredients which do not affect the end result may be added. The products, compositions, methods and processes of the present invention can include all the essential elements and limitations of the invention described herein as well as any of the additional or optional ingredients, components, steps, or limitations described herein.

"Free" or "substantially free" or "devoid of" as it is used herein means that while it is preferred that no amount of the specific component be present in the composition, it is possible to have very small amounts of it in the compositions of the invention provided that these amounts do not materially affect at least one, preferably most, of the advantageous properties of the conditioning compositions of the invention. Thus, for example, "free of phenylated solvents" means that non-volatile solvents are preferably omitted (that is 0% by weight), but can be present in the composition at an amount of less than about 0.25% by weight, typically less than about 0.1% by weight, typically less than about 0.05% by weight, based on the total weight of the composition as a whole.

As used herein, all ranges provided are meant to include every specific range within, and combination of subranges between, the given ranges. Thus, a range from 1-5, includes specifically 1, 2, 3, 4 and 5, as well as subranges such as and 2-5, 3-5, 2-3, 2-4, 1-4, etc.

As used herein a range of ratios is meant to include every specific ratio within, and combination of subranges between the given ranges.

"Keratinous materials" includes materials containing keratin such as hair, skin, eyebrows, lips and nails.

"Volatile", as used herein, means having a flash point of less than about 100°C.

"Non-volatile", as used herein, means having a flash point of greater than about 100°C.

The composition of the present invention may be in any form, either liquid or non-liquid (semi-solid, soft solid, solid, etc.). For example, it may be a paste, a solid, a gel, or a cream. It may be an emulsion, such as an oil-in-water or water-in-oil emulsion, a multiple emulsion, such as an oil-in-water-in-oil emulsion or a water-in-oil-in-water emulsion, or a solid, rigid or supple gel. The composition of the invention may, for example, comprise an external or continuous fatty phase. The composition can also be a molded composition or cast as a stick or a dish.

The compositions and methods of the present invention can comprise, consist of, or consist essentially of the essential elements and limitations of the invention described herein, as well as any additional or optional ingredients, components, or limitations described herein or otherwise useful.

### Long-Wear Lip Composition

According to embodiments of the present invention, a long-wear lip composition comprising at least one silicone film-forming agent is provided. Preferably, the silicone film-forming agent is selected from the group consisting of silicone resins, polyorganosiloxane copolymers, and mixtures thereof.

### Silicone Resins

According to preferred embodiments, the long-wear lip compositions of the present invention comprise at least one silicone resin. Examples of suitable silicone resins include those described, for example, in U.S. Pat. No. 5,505,937, U.S. Pat. No. 5,911,974, U.S. Pat. No. 5,965,112, U.S. Pat. No. 5,985,298, U.S. Pat. No. 6,074,654, U.S. Pat. No. 6,780,422, U.S. Pat. No. 6,908,62 1.

According to preferred embodiments, the long-wear lip compositions contain siloxysilicate resins. One non-limiting example of a siloxysilicate in accordance with the present invention is trimethylsiloxysilicate, which may be represented by the following formula:

[(CH₃)₃SiO]ₓ(SiO_{4/2})_{y}

wherein x and y may, for example, range from 50 to 80. Such siloxysilicates are commercially available from General Electric, Dow Corning, Wacker, Milliken, Siltech, Grant Industries, Momentive and Shin-Etsu Silicones under the tradename Resin MQ^{®}.

According to preferred embodiments, the long-wear lip compositions contain silsesquioxane resins such as, for example, polypropyl silsesquioxane resin.

Silsesquioxane resins are a specific form of silicone resin. Silicone resin nomenclature is known in the art as "MDTQ" nomenclature, whereby a silicone resin is described according to the various monomeric siloxane units which make up the polymer. Each letter of "MDTQ" denotes a different type of unit. When the film-forming resin is made up predominantly of tri-functional units (or T units), it is generally called a silsesquioxane resin, which is described, for example in US 2006/0292096.

Examples of silsesquioxane resins that may be used in the present invention are alkyl silsesquioxane resins that are silsesquioxane homopolymers and/or copolymers having an average siloxane unit of the general formula R¹ₙ SiO_{(4-n)/2}, wherein each R1 is a propyl group, wherein more than 80 mole % of R1 represent a C3 -C10 alkyl group, n is a value of from 1.0 to 1.4, and more than 60 mole % of the copolymer comprises R¹SiO_{3/2} units. As each R1 is a propyl group these polymers are called polypropylsilsesquioxane resins or "t-propyl" silsesquioxane resins. These resins and methods of making them are described, for example in US 8,586,013, 2012/0301415, 2007/0093619, and 2006/0292096.

A non-limiting example of a polypropylsilsesquioxane resin suitable for use in the present invention is commercially available from Dow Corning as Dow Corning 670 Fluid or Dow Corning 680 Fluid. These Dow Corning resins have a general formula of RₙSiO_{(4-n)/2} wherein R is independently chosen from a hydrogen atom and a monovalent hydrocarbon group comprising 3 carbon atoms, wherein more than 80 mole % of R are propyl groups, n is a value from 1.0 to 1.4, more than 60 mole % of the copolymer comprises RSiO_{3/2} units, and having a hydroxyl or alkoxy content from 0.2 to 10% by weight, for example between 1 and 4% by weight, preferably between 5 and 10% by weight, and more preferably between 6 and 8% by weight. Preferably, the polypropylsilsesquioxane resin has a molecular weight from about 5000 to about 30,000 and a Tg from about -5°C to about 5°C.

According to preferred embodiments, the long-wear lip composition contains at least one silicone resin selected from the group consisting of siloxysilicate resins, silsesquioxane resins, and mixtures thereof.

The at least one silicone resin is preferably present in the long-wear lip compositions of the present invention in an amount ranging from about 5% to about 30% by weight, preferably from about 10% to about 25% by weight, and preferably from about 15% to about 20% by weight, including all ranges and subranges therebetween, all weights being based on the weight of the composition as a whole.

### Polyorganosiloxane Copolymer

According to preferred embodiments, the long-wear lip compositions of the present invention comprise at least one polyorganosiloxane copolymer. The polyorganosiloxane copolymer useful herein is preferably a polymer (homopolymer or copolymer) having at least one moiety which contains: at least one polyorganosiloxane group consisting of 1 to about 1000 organosiloxane units in the chain of the moiety or in the form of a graft, and at least two groups capable of establishing hydrogen interactions. Non-limiting examples of polyorganosiloxane copolymers are disclosed, for example in US. Pat. No. 8,945,525.

Additional polyorganosiloxane copolymers which may be used in the long-wear lip compositions of the present invention include those described in documents US 5,874,069, US 5,919,441, US 6,051,216, and US 5,981,680.

A preferred polyorganosiloxane copolymer for use in the present invention contain at least one moiety chosen from formula (III): 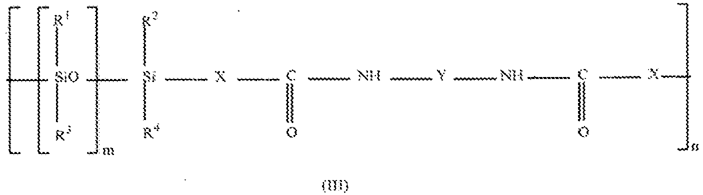 and formula (IV) 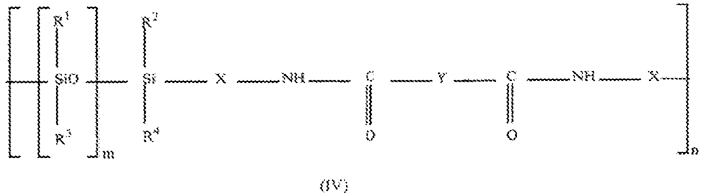 in which:
(a) R¹, R², R³ and R⁴ are the same or different and may be selected from the group consisting of methyl, ethyl, propyl, isopropyl, a siloxane chain, and phenyl;
(b) X is a linear or branched chain alkylene having 1-30 carbons;
(c) Y is selected from the group consisting of linear or branched chain alkylenes having 1-40 carbons;
(d) m is a number between 1 and 700;
(e) n is a number between 1 and 500.

Particularly preferred polyorganosiloxane copolymers useful herein are commercially available from Dow Corning under the tradenames DC 8178^{®} and DC 8179^{®}, which are known under the INCI denomination of Nylon-611/Dimethicone Copolymer.

Preferably, the polyorganosiloxane copolymer is present in the long-wear lip compositions of the present invention in an amount ranging from about 2% to about 35% by weight, preferably from about 5% to about 30% by weight, and preferably from about 7% to about 20% by weight, including all ranges and subranges therebetween, all weights being based on the weight of the composition as a whole.

### Silicone Elastomer (silicone crosspolymer)(optional)

According to preferred embodiments, the long-wear lip composition further comprises as least one silicone elastomer. Preferably, the silicone elastomer is a non-emulsifying silicon elastomer. "Non-emulsifying" defines organopolysiloxane elastomers that do not contain hydrophilic chains, in particular polyoxyalkylene (especially polyoxyethylene or polyoxypropylene) or polyglyceryl units. Thus, according to one particular embodiment of the invention, the composition comprises a silicone elastomer that is free of polyoxyalkylene units and polyglyceryl units.

Suitable examples of non-emulsifying elastomers are described in US 8,637,057.

Further suitable examples of non-emulsifying elastomers useful in this invention include but not limit those sold under the names KSG-6, KSG-15, KSG-16, KSG-18, KSG-41, KSG-42, KSG-43 and KSG-44 by the company Shin-Etsu, DC 9040, DC 9041, DC 9509, DC 9505 and DC 9506, by the company Dow Corning, and SFE 839 by the company General Electric.

The silicone elastomers are preferably in the form of a gel or a powder.

According to preferred embodiments, the silicone elastomer particles are conveyed in the form of a gel formed from an elastomeric organopolysiloxane included in at least one hydrocarbon-based oil and/or one silicone oil. In these gels, the organopolysiloxane particles are often non-spherical particles. Not limiting examples of silicone elastomers useful in this invention are dimethicone crosspolymer gels (blends of dimethicone crosspolymers in solvents) having viscosity values from about 150 and to about 700 mm²/s, from about 200 to about 650 mm²/s and from about 300 to about 600 mm²/s, including all ranges and subranges therebetween.

Particularly useful for this invention may be blends of high molecular weight silicone elastomers in volatile solvents, such as silicone oils, hydrocarbon oils and mixtures thereof.

Specific examples of suitable silicone elastomeric gels include DC EL-8040 ID (INCI name: Isododecane (and) Dimethicone Crosspolymer) and DC EL-9140 DM (INCI name: Dimethicone (and) Dimethicone Crosspolymer), supplied by Dow Corning.

Non-limiting examples of silicone elastomers and their synthesis are disclosed, for example in U.S. 8, 637,057 and US/20150174048.

Although not wishing to be bound by any particular theory, it is believed that the silicone elastomer thickens the composition, adds the cushiony (spongy) effect and/or improves the application properties of the long-wear lip composition. Also, it provides a very soft feel and/or mattifying effect after application.

Preferably, the silicone elastomer is present in a content ranging from 1 percent to 30 percent by weight of active material (dry matter), preferably from about 1.5 percent to about 20 percent, and preferably from 2 percent to 10 percent by weight relative to the total weight of the long-wear lip composition, including all ranges and subranges therebetween.

### Topcoat Composition

According to the present invention, topcoat compositions comprising at least one phenylated silicone oil and at least one wax are provided. Preferably, the topcoat composition consists essentially of at least one phenylated silicone oil and at least one wax. Preferably, the topcoat composition does not comprise diglyceryl polyacyladipate. Preferably, when the topcoat composition is applied to a long-wear lip composition, the topcoat composition does not inhibit the transfer-resistance of the long-wear lip composition.

According to preferred embodiments of the present invention, the topcoat composition does not comprise diglyceryl polyacyladipate in an amount which inhibits the transfer-resistance of the long-wear lip composition to which it has been applied, it will be applied, and/or after application. Also preferably, the topcoat composition does not comprise:
(1) synthetic esters of formula R₁COOR₂ in which R₁ represents the residue of a linear or branched higher fatty acid containing from 7 to 40 carbon atoms and R₂ represents a branched hydrocarbon-based chain containing from 3 to 40 carbon atoms, for instance, purcellin oil (cetostearyl octanoate), isononyl isononanoate, C12 to C15 alcohol benzoate, 2-ethylhexyl palmitate, 2-octyldodecyl stearate, 2-octyldodecyl erucate, isostearyl isostearate, 2-octyldodecyl benzoate, octanoates, decanoates or ricinoleates of alcohols or of polyalcohols, isopropyl myristate, isopropyl palmitate, butyl stearate, hexyl laurate, diisopropyl adipate, 2-ethylhexyl palmitate, 2-hexyldecyl laurate, 2-octyldecyl palmitate, 2-octyldodecyl myristate, 2-diethylhexyl succinate, diisostearyl malate, isodecyl neopentanoate, hydroxylated esters such as isostearyl lactate, octyl hydroxy stearate, octyldodecyl hydroxystearate, diisostearyl malate, triisocetyl citrate, glyceryl or diglyceryl triisostearate; diethylene glycol diisononanoate; pentaerythritol esters; esters of aromatic acids and of alcohols containing from 4 to 22 carbon atoms, in particular tridecyl trimellitate;
(2) C8-C26 higher fatty acids such as myristic acid, oleic acid, linoleic acid, linolenic acid or isostearic acid; and/or
(3) C8-C26 higher fatty alcohols such as oleyl alcohol, linoleyl alcohol, linolenyl alcohol, isostearyl alcohol or octyldodecanol;
in an amount which inhibits the transfer-resistance of the long-wear lip composition to which it has been applied, it will be applied, and/or after application.

### Phenylated Silicone Oil

According to preferred embodiments, the topcoat composition of the present invention comprises at least one phenylated silicone oil.

Suitable phenylated silicone oils include, but are not limited to, non-volatile phenylated silicone oils such as, for example, linear or branched non-volatile polydimethylsiloxanes (PDMS) comprising phenyl groups which are pendent or at the end of the silicone chain.

Suitable examples include, but are not limited to, phenyl trimethicones, such as phenyl trimethylsiloxy trisiloxane sold under the reference Dow Corning 556 Cosmetic Grade Fluid, phenyl dimethicones, phenyl trimethylsiloxy diphenylsiloxanes, diphenyl dimethicones, diphenyl methyldiphenyl trisiloxanes, 2-phenylethyl trimethylsiloxysilicates, trimethyl pentaphenyl trisiloxane, such as the silicone oil sold by Dow Corning under the reference PH-1555 HRI or Dow Corning 555 Cosmetic Fluid (chemical name: 1,3,5-trimethyl-1,1,3,5,5-pentaphenyl trisiloxane; INCI name: trimethyl pentaphenyl trisiloxane), and trimethyl siloxyphenyl dimethicones, such as the product sold under the reference Belsil PDM 1000 by the company Wacker.

Preferably, phenylated silicone oil is present in the topcoat compositions of the present invention in an amount ranging from about 50% to about 96% by weight, from about 60% to about 96% by weight, from about 70% to 95% by weight, and preferably from about 75% to about 95% by weight, including all ranges and subranges therebetween, all weights based on the weight of the composition as a whole.

### Wax

According to preferred embodiments, the topcoat composition of the present invention comprises at least one wax. For the purposes of the present invention, a wax is a lipophilic fatty compound that is solid at room temperature (25°C), has a reversible solid/liquid change of state (that is, the state of the material may change based on temperature), has a melting point greater than 45°C, preferably greater than 55°C, more preferably between about 65°C to about 120°C, and has anisotropic crystal organization in the solid state. The melting point of the wax may be measured using a differential scanning calorimeter (DSC), for example the calorimeter sold under the name DSC 30 by Mettler. For waxes that are derived from petroleum, such as microcrystalline wax, the melting point may be measured according to the drop ASTM method, D-127.

The waxes can generally be those used in cosmetics. The waxes may be of natural origin, for instance beeswax, carnauba wax, candelilla wax, ouricoury wax, Japan wax, cork fiber wax, sugar cane wax, paraffin wax, lignite wax, microcrystalline waxes, lanolin wax, montan wax, ozokerites and hydrogenated oils, for instance hydrogenated jojoba oil.

The waxes also may be of synthetic origin, for instance polyethylene waxes derived from the polymerization of ethylene, waxes obtained by Fischer-Tropsch synthesis, esters of fatty acids and of glycerides that are solid at 40°C.

Particular waxes include, for example, polyethylene waxes, for example the product sold under the name Performalene 500-L Polyethylene (New Phase Technology), and polymethylene waxes, for instance the product sold under the name Cirebelle 303 (Sasol).

Particular waxes include, for example, silicone waxes such as polypropylsilsesquioxane wax substituted with alkyl units having at least 30 carbons. Polypropylsilsesquioxane waxes, in general, have been disclosed in patent publication WO2005/100444 and U.S. 8,586,013.

A particularly preferred polypropylsilsesquioxane wax for use in the present invention is a C30-45 ALKYLDIMETHYLSIL YL POLYPROPYLSILSESQUIOXANE commercially available from DOW CORNING under the tradename SW-8005 C30 Resin Wax.

According to preferred embodiments, the topcoat composition comprises at least one wax selected from the group consisting of beeswax, ozokerite, and mixtures thereof.

According to preferred embodiments, the topcoat composition comprises at least one wax selected from the group consisting of beeswax, paraffin wax, microcrystalline wax, synthetic wax, and mixtures thereof.

Preferably, wax is present in the topcoat compositions of the present invention in an amount ranging from about 1% to about 30% by weight, from about 3% to about 25% by weight, from about 4% to 40% by weight, and preferably from about 5% to about 15% by weight, including all ranges and subranges therebetween, all weights based on the weight of the composition as a whole.

### Additional Ingredients

According to preferred embodiments, the long-wear lip composition, the topcoat composition, or both can comprise additional ingredients typically included in cosmetic compositions. A non-exhaustive discussion of such ingredients is set forth below.

### Film-Forming Agents

According to preferred embodiments, the topcoat composition further comprises as least one film-forming agent, the long-wear lip composition further comprises an additional film-forming agent other than the at least one silicone film-forming agent discussed above, or both. Preferably, if a film-forming agent is present in the topcoat composition, the film-forming agent is a silicone film-forming agent selected from the group consisting of silicone resins, polyorganosiloxane copolymers, and mixtures thereof. Also preferably, the silicone resin is selected from the group consisting of siloxysilicate resins, silsesquioxane resins, and mixtures thereof. The discussion above concerning silicone film-forming agents in the long-wear lip composition applies to the silicone film-forming agent in the topcoat composition as well.

### Volatile Solvent

The compositions of the invention may optionally further comprise at least one volatile solvent.

The expression "volatile solvent" means any non-aqueous medium capable of evaporating on contact with the skin or the lips in less than one hour at room temperature and atmospheric pressure.

Examples of suitable volatile solvents include volatile hydrocarbon-based oils such as, for example, volatile hydrocarbon oils having from 8 to 16 carbon atoms and their mixtures and in particular branched C₈ to C₁₆ alkanes such as C₈ to C₁₆ isoalkanes (also known as isoparaffins), isododecane, isodecane, isohexadecane, and for example, the oils sold under the trade names of Isopar or Permethyl, the C₈ to C₁₆ branched esters such as isohexyl or isodecyl neopentanoate, alcohols, and their mixtures. Preferably, the volatile hydrocarbon-based oils have a flash point of at least 40°C.

Examples of volatile hydrocarbon-based oils include, but are not limited to those given in Table 1 below.

**Table 1**

| Compound | Flash Point (°C) |
|---|---|
| Isododecane | 43 |
| Isohexadecane | 102 |
| Isodecyl neopentanoate | 118 |
| Propylene glycol n-butyl ether | 60 |
| Ethyl 3-ethoxypropi onate | 58 |
| Propylene glycol methylether acetate | 46 |
| Isopar L (isoparaffin C₁₁-C₁₃) | 62 |
| Isopar H (isoparaffin C₁₁-C₁₂) | 56 |

The volatile solvent may also be chosen from volatile silicone oils, which may be linear or cyclic, having a viscosity, at room temperature, of less than or equal to 6 cSt, and having from 2 to 7 silicon atoms, optionally substituted with alkyl or alkoxy groups of 1 to 10 carbon atoms.

Examples of suitable volatile silicone oils include, but are not limited to, those listed in Table 2 below.

**Table 2**

| Compound | Flash Point (°C) | Viscosity (cSt) |
|---|---|---|
| Octyl trimethicone | 93 | 1.2 |
| Hexyltrimethicone | 79 | 1.2 |
| Decamethylcyclopentasiloxane (cyclopentasiloxane or D5) | 72 | 4.2 |
| Octamethylcyclotetrasiloxane (cyclotetradimethylsiloxane or D4) | 55 | 2.5 |
| Dodecamethylcyclohexasiloxane (D6) | 93 | 7 |
| Decamethyltetrasiloxane(L4) | 63 | 1.7 |
| KF-96 A from Shin Etsu | 94 | 6 |
| PDMS (polydimethylsiloxane) DC 200 (1.5cSt) from Dow Corning | 56 | 1.5 |
| PDMS DC 200 (2cSt) from Dow Corning | 87 | 2 |
| PDMS DC 200 (5cSt) from Dow Corning | 134 | 5 |
| PDMS DC 200 (3St) from Dow Corning | 102 | 3 |

The at least one volatile solvent, if present, is generally present in the compositions of the present invention in an amount ranging from about 5% to about 50% by weight; such as from about 10% to about 45% by weight; such as from about 15% to about 40% by weight, all weights being based on the weight of the composition as a whole.

### Non-volatile Solvent

The compositions of the present invention may optionally further comprise at least one non-volatile solvent (oil).

The volatility of the oils can be determined using the evaporation speed as set forth in U.S. Pat. No. 6,338,839.

Non-volatile oils include low viscosity oils (having a viscosity from about 5 to about 10 centipoise) and high viscosity oils (having a viscosity of from about 100 to about 10,000 centipoise), and mixtures thereof. In contrast to waxes, oils are liquids at room temperature.

According to a particular embodiment of the present invention, the oil is a high viscosity oil which is a silicone oil and/or a hydrocarbon oil. "High viscosity" means an oil having a viscosity greater than 100 cSt, particularly greater than 250 cSt at 25°C. Most particularly, the non-volatile oil is selected from a silicone oil. Such oils are described, for example in US 2011/0293550 and US 2004/0126350.

Non-limiting examples of suitable non-volatile silicone oils include polymethylsiloxanes (PDMS) with a linear or cyclic silicone chain, which are liquid or pasty at room temperature, especially cyclopolydimethylsiloxanes (cyclomethicones) such as cyclohexasiloxane; polydimethyl-siloxanes (CTFA designation "dimethicones") comprising alkyl or alkoxy groups, which are pendent or at the end of a silicone chain, these groups containing from 2 to 24 carbon atoms; polydiethyl siloxanes; and dimethicone fluids having viscosity from about 300 cPs at 25°C to about 1500 cPs at 25°C. Particularly useful dimethicone fluids have viscosity from about 350 cPs at 25°C to about 1000 cPs at 25°C.

Specific examples of suitable for this invention high viscosity silicone oils include, but are not limited to, Xiameter^{®} silicone fluids from Dow Coming.

The at least one non-volatile silicone oil, if present, is preferably present in the compositions of the present invention in an amount ranging from about 2% to about 30% by weight, including from about 4% to about 25% by weight, typically about 6% to about 20% by weight, based on the total weight of the composition, including all ranges and subranges within these ranges.

According to preferred embodiments of the long-wear lip composition, combining at least one silicone elastomer, preferably at least one dimethicone crosspolymer, and at least one non-volatile silicone fluid having a viscosity greater than or equal to 350 cSt at 25°C, in a ratio where the weight of the silicone elastomer to the weight of the non-volatile silicone fluid is higher or equal to from about 1:0.02 and is lower or equals to from about 1:10 by weight (for example, 1:0.02 to 1:10), including all ranges and subranges therebetween such as, for example, from about 1:1 to about 1:6 and from about 1:1 to about 1:5, yields a cosmetic characterized by long wear, transfer resistance, non-tackiness, limited flaking and great comfort.

According to other preferred embodiments of the long-wear lip composition, combining at least one silicone elastomer, preferably at least one dimethicone crosspolymer, and at least one non-volatile silicone fluid having a viscosity greater than or equal to 350 cSt at 25°C, in a ratio where the weight of the silicone elastomer to the weight of the non-volatile silicone fluid is higher or equal to from about 1:0.02 and is lower or equals to from about 1:10 by weight (for example, 1:0.02 to 1:10), including all ranges and subranges therebetween such as, for example, from about 1:1 to about 1:6 and from about 1:1 to about 1:5, yields a cosmetic characterized by long wear, transfer resistance, non-tackiness, limited flaking and great comfort.

### Wax

The long-wear lip compositions of the present invention may optionally further comprise at least one wax. The discussion above concerning wax in the topcoat compositions applies to the wax in the long-wear lip composition as well.

### Pigments

The compositions of the present invention may optionally further comprise at least one cosmetically acceptable colorant such as a pigment or dyestuff. Examples of suitable pigments include, but are not limited to, inorganic pigments, organic pigments, lakes, pearlescent pigments, iridescent or optically variable pigments, and mixtures thereof. A pigment should be understood to mean inorganic or organic, white or colored particles. Said pigments may optionally be surface-treated within the scope of the present invention but are not limited to treatments such as silicones, perfluorinated compounds, lecithin, and amino acids.

Representative examples of inorganic pigments useful in the present invention include those selected from the group consisting of rutile or anatase titanium dioxide, coded in the Color Index under the reference CI 77,891; black, yellow, red and brown iron oxides, coded under references CI 77,499, 77, 492 and, 77,491; manganese violet (CI 77,742); ultramarine blue (CI 77,007); chromium oxide (CI 77,288); chromium hydrate (CI 77,289); and ferric blue (CI 77,510) and mixtures thereof.

Representative examples of organic pigments and lakes useful in the present invention include, but are not limited to, D&C Red No. 19 (CI 45,170), D&C Red No. 9 (CI 15,585), D&C Red No. 21 (CI 45,380), D&C Orange No. 4 (CI 15,510), D&C Orange No. 5 (CI 45,370), D&C Red No. 27 (CI 45,410), D&C Red No. 13 (CI 15,630), D&C Red No. 7 (CI 15,850), D&C Red No. 6 (CI 15,850), D&C Yellow No. 5 (CI 19,140), D&C Red No. 36 (CI 12,085), D&C Orange No. 10 (CI 45,425), D&C Yellow No. 6 (CI 15,985), D&C Red No. 30 (CI 73,360), D&C Red No.3 (CI 45,430) and the dye or lakes based on cochineal carmine (CI 75,570) and mixtures thereof.

Representative examples of pearlescent pigments useful in the present invention include those selected from the group consisting of the white pearlescent pigments such as mica coated with titanium oxide, mica coated with titanium dioxide, bismuth oxychloride, titanium oxychloride, colored pearlescent pigments such as titanium mica with iron oxides, titanium mica with ferric blue, chromium oxide and the like, titanium mica with an organic pigment of the above-mentioned type as well as those based on bismuth oxychloride and mixtures thereof.

The precise amount and type of colorant employed in the compositions of the present invention will depend on the color, intensity and use of the cosmetic composition and, as a result, will be determined by those skilled in the art of cosmetic formulation. However, one preferred amount of colorant for use in the present invention is from about 0.5% to about 15%, from about 1.5% to about 12%, and from about 2% to about 10%, based on the weight of the composition.

### Filler

The compositions of the present invention may optionally further comprise at least one filler. Fillers that may be used in the compositions of the invention include, for example, silica powder; talc; polyamide particles and especially those sold under the name Orgasol by the company Atochem; polyethylene powders; microspheres based on acrylic copolymers, such as those based on ethylene glycol dimethacrylate/lauryl methacrylate copolymer sold by the company Dow Corning under the name Polytrap; expanded powders such as hollow microspheres and especially the microspheres sold under the name Expancel by the company Kemanord Plast or under the name Micropearl F 80 ED by the company Matsumoto; powders of natural organic materials such as crosslinked or noncrosslinked corn starch, wheat starch or rice starch, such as the powders of starch crosslinked with octenyl succinate anhydride, sold under the name Dry-Flo by the company National Starch; silicone resin microbeads such as those sold under the name Tospearl by the company Toshiba Silicone; clays (bentone, laponite, saponite, etc.); and mixtures thereof.

The fillers may be present in the composition of the invention in an amount ranging from about 0.1% to about 50% by weight, such as from 0.5% to about 30% by weight, and such as from about 1% to about 20% by weight, all weights based on the weight of the composition as a whole.

### Silica Aerogels

The compositions of the present invention may optionally further comprise at least one silica aerogel. Silica aerogels are porous materials obtained by replacing (by drying) the liquid component of a silica gel with air. They are generally synthesized via a sol-gel process in liquid medium and then dried, usually by extraction of a supercritical fluid, the one most commonly used being supercritical CO₂. This type of drying makes it possible to avoid shrinkage of the pores and of the material. The sol-gel process and the various drying processes are described in detail in Brinker CJ., and Scherer G.W., Sol-Gel Science: New York: Academic Press, 1990. Silica aerogels, in general, have been disclosed in U.S. Pat. No. 9,320,689.

As hydrophobic silica aerogels that may be used in the invention, examples that may be mentioned include the aerogel sold under the name VM-2260 (INCI name: Silica silylate), by the company Dow Corning, the particles of which have a mean size of about 1000 microns and a specific surface area per unit of mass ranging from 600 to 800 m²/g.

Mention may also be made of the aerogels sold by the company Cabot under the references AEROGEL TLD 201, AEROGEL OGD 201, AEROGEL TLD 203, ENOVA^{®} AEROGEL MT 1100, ENOVA AEROGEL MT 1200.

In particular, the aerogel sold under the name VM-2270 (INCI name: Silica silylate), by the company Dow Corning, the particles of which have a mean size ranging from 5-15 microns and a specific surface area per unit of mass ranging from 600 to 800 m²/g can be used.

The silica aerogel particles can be used in the inventive compositions from 0.1% to about 8% by weight, preferably from 0.25% to 6% by weight, and preferably from 0.5% to 4% by weight, including all ranges and subranges therebetween, all weights based on the weight of the composition as a whole.

### Additional Additives

The compositions of the present invention may optionally further comprise at least one cosmetically or dermatologically acceptable additive such as thickener, a plasticizer, an antioxidant, an essential oil, a botanical extract, a fragrance, a preserving agent, a fragrance, a pasty fatty substance, a neutralizing agent, and a polymer, and cosmetically active agents and/or dermatological active agents such as, for example, emollients, moisturizers, vitamins, essential fatty acids and medicaments.

According to preferred embodiments, the long-wear lip composition is a composition comprising:
(a) from about 1% to about 30% by weight of at least one silicone elastomer;
(b) from about 2% to about 30% by weight of at least one non-volatile oil;
(c) from about 2% to about 35% by weight of at least one polyorganosiloxane copolymer;
(d) from about 5% to about 30% by weight of at least one silicone resin;
(e) from about 5% or about 50% of at least one volatile solvent;
(f) optionally at least one wax;
(g) optionally at least one colorant; and
(h) optionally at least one filler;
wherein the ratio of the silicone elastomer (a) to the at least one non-volatile oil (b) is from about 1:0.02 to about 1:10; the weights being relative to the total weight of the composition.

According to preferred embodiments, the long-wear lip composition is a liquid composition comprising:
a) from about 1% to about 30% by weight of at least one dimethicone crosspolymer ;
b) from about 2% to about 30% by weight of at least one non-volatile silicone oil having viscosity greater than or equal to 350 cSt;
c) from about 2% to about 35% by weight of Nylon-611/Dimethicone copolymer;
d) from about 5% to about 30% by weight of at least one siloxysilicate resin;
e) from about 5% or about 50% of at least one volatile hydrocarbon solvent;
f) optionally at least one wax;
g) optionally at least one colorant; and
h) optionally at least one filler;
wherein the ratio of the silicone elastomer (a) to the at least one non-volatile oil (b) is from about 1:0.02 to about 1:10; the weights being relative to the total weight of the composition.

According to preferred embodiments, kits comprising (1) a long-wear lip composition comprising at least one silicone film-forming agent, and (2) a topcoat composition comprising at least one phenylated silicone oil and at least one wax are provided. Preferably, the topcoat composition consists essentially of at least one phenylated silicone oil and at least one wax. Preferably, the topcoat composition does not comprise diglyceryl polyacyladipate. Preferably, when the topcoat composition is applied to the long-wear lip composition, the topcoat composition does not inhibit the transfer-resistance of the long-wear lip composition.

According to preferred embodiments, cosmetic systems comprising (1) a long-wear lip composition comprising at least one silicone film-forming agent, and (2) a topcoat composition comprising at least one phenylated silicone oil and at least one wax are provided. Preferably, the topcoat composition consists essentially of at least one phenylated silicone oil and at least one wax. Preferably, the topcoat composition does not comprise diglyceryl polyacyladipate. Preferably, when the topcoat composition is applied to the long-wear lip composition, the topcoat composition does not inhibit the transfer-resistance of the long-wear lip composition.

According to preferred embodiments, methods for enhancing the appearance of lips comprising applying a long-wear lip composition comprising at least one silicone film-forming agent to the lips, and (2) applying a topcoat composition comprising at least one phenylated silicone oil and at least one wax to the long-wear lip composition are provided. Preferably, the topcoat composition consists essentially of at least one phenylated silicone oil and at least one wax. Preferably, the topcoat composition does not comprise diglyceryl polyacyladipate. Preferably, when the topcoat composition is applied to the long-wear lip composition, the topcoat composition does not inhibit the transfer-resistance of the long-wear lip composition.

According to preferred embodiments, methods for making-up lips comprising applying a long-wear lip composition comprising at least one silicone film-forming agent to the lips, and (2) applying a topcoat composition comprising at least one phenylated silicone oil and at least one wax to the long-wear lip composition are provided. Preferably, "making up" the lips includes applying at least one coloring agent to the lips in an amount sufficient to provide color to the lips. Preferably, the topcoat composition consists essentially of at least one phenylated silicone oil and at least one wax. Preferably, the topcoat composition does not comprise diglyceryl polyacyladipate. Preferably, when the topcoat composition is applied to the long-wear lip composition, the topcoat composition does not inhibit the transfer-resistance of the long-wear lip composition.

According to preferred embodiments, methods for treating lips comprising applying a long-wear lip composition comprising at least one silicone film-forming agent to the lips, and (2) applying a topcoat composition comprising at least one phenylated silicone oil and at least one wax to the long-wear lip composition are provided. Preferably, the topcoat composition consists essentially of at least one phenylated silicone oil and at least one wax. Preferably, the topcoat composition does not comprise diglyceryl polyacyladipate. Preferably, when the topcoat composition is applied to the long-wear lip composition, the topcoat composition does not inhibit the transfer-resistance of the long-wear lip

The compositions and methods of the present invention can "comprise," "consist of" or "consist essentially of" the identified ingredients and process steps. For purposes of the compositions and methods of the present invention where the invention "consists essentially of" the identified ingredients and/or process steps, the sole "basic and novel property" of such compositions and/or methods is transfer-resistance. Further, given that it is contemplated that other transfer-resistance enhancers or boosters (for example, additional film-forming agents) can be added to the invention methods and compositions in the context of the present invention, a "material effect" on the basic and novel property of the invention can only be an adverse effect. That is, because positive effects on transfer-resistance properties (such as those effected by additional film-forming agents) are within the scope of the present invention, only ingredients which have a material adverse effect on transfer-resistance properties would be relevant to determining whether or not compositions or methods "consist essentially of" the required elements.

Unless otherwise indicated, all numbers expressing quantities of ingredients, reaction conditions, and so forth used in the specification and claims are to be understood as being modified in all instances by the term "about." Accordingly, unless indicated to the contrary, the numerical parameters set forth in the following specification and attached claims are approximations that may vary depending upon the desired properties sought to be obtained by the present invention.

Notwithstanding that the numerical ranges and parameters setting forth the broad scope of the invention are approximations, the numerical values set forth in the specific examples are reported as precisely as possible. Any numerical value, however, inherently contain certain errors necessarily resulting from the standard deviation found in their respective measurements. The following examples are intended to illustrate the invention without limiting the scope as a result. The percentages are given on a weight basis.

### EXAMPLES

The present invention will be better understood from the examples which follow. The examples are intended to be nonrestrictive and explanatory only, with the scope of the invention defined by the claims.

### Example 1 - Invention Compositions

The following tables set forth exemplary topcoat compositions

| Ingredient | Composition 1 | Invention Composition 2 |
|---|---|---|
| TRIMETHYL PENTAPHENYL TRISILOXANE | 90.75 | 80.75 |
| OZOKERITE | 6.3 | 6.3 |
| BEESWAX | 2.7 | 2.7 |
| TOCOPHERYL ACETATE | 0.2 | 0.2 |
| FRAGRANCE | 0.05 | 0.05 |
| SUCROSE ACETATE ISOBUTYRATE | --- | 5.0 |
| PHENYLPROPYLDIMETHYLSILOXYSILICATE | --- | 5.0 |

| INCI US Name | Composition 3 | Invention Composition 4 | Invention Composition 5 | Invention Composition 6 |
|---|---|---|---|---|
| TRIMETHYL PENTAPHENYL TRISILOXANE | 90.75 | 80.75 | 80.75 | 79.75 |
| OZOKERITE | 6.3 | 6.3 | 0 | 0 |
| PARAFFIN (and) MICROCRYSTALLINE WAX (and) SYNTHETIC WAX | 0 | 0 | 6.3 | 7 |
| BEESWAX | 2.7 | 2.7 | 2.7 | 3 |
| TOCOPHERYL ACETATE | 0.2 | 0.2 | 0.2 | 0.2 |
| FRAGRANCE | 0.05 | 0.05 | 0.05 | 0.05 |
| SUCROSE ACETATE ISOBUTYRATE | 0 | 5 | 5 | 5 |
| PHENYLPROPYLDIMETHYLSILOXYSILICATE | 0 | 5 | 5 | 5 |

### Example 2 - Exemplary Method Of Preparing Invention Composition(s)

Sucrose acetate isobutyrate, phenylpropyldimethylsiloxysilicate, and trimethyl pentaphenyl trisiloxane were mixed in a container at 90°C until a clear liquid was obtained. Ozokerite and beeswax were added to the mixture and stirred at 90°C until the waxes melted and the solution was clear. Tocopheryl acetate and fragrance were then added. The resulting mixture was poured into a metal mold and cooled to room temperature, followed by refrigeration for 10 minutes. Sticks were removed from the mold and placed into appropriate packs.

### Example 3 - Exemplary Long-Wear Lip Composition

| Ingredients | Inventive Compositions (% weight) |
|---|---|
| MQ | 17.55 |
| PSPA (nylon 611/dimethicone) | 9.78 |
| 1000cst Dimethicone fluid | 11 |
| Lauryl lysine | 1.5 |
| Pigment blend | 6.41 |
| Silicone resin wax | 0.5 |
| 9140 dm Silicone elastomer (14% active) | 35.71 (5%) |
| Fillers | 3.33 |
| Fragrance | 0.1 |

| Isododecane | QS |
|---|---|
| Ratio of silicone elastomer ( % active) : dimethicone | 1:2.2 |

### Example 4 - Commercial Long-Wear Lip Composition And Comparative Commercial Topcoat

A commercial product having the identified basecoat and topcoat was evaluated. The basecoat of the commercial product is an acceptable long-wear lip composition in accordance with the present invention. The topcoat of the commercial product is not an acceptable topcoat, and it does not constitute part of the present invention.

Basecoat (long-wear lip composition): isododecane, trimethylsiloxysilicate, nylon-611/dimethicone copolymer, disteardimonium hectorite, lauroyl lysine, c30-45 alkyldimethylsilyl polypropylsilsesquioxane, alumina, propylene carbonate, synthetic fluorphlogopite, silica, calcium sodium borosilicate, calcium aluminum borosilicate, polyethylene terephthalate, fragrance, aluminum hydroxide, acrylates copolymer, benzyl alcohol, dimethicone, paraffin, tin oxide. [+/- may contain mica, ci 77891/titanium dioxide, ci 77491, ci 77492, ci 77499/iron oxides, ci 15850/red 7, ci 15985/yellow 6 lake, ci 45410/red 28 lake, ci 45380/red 22 lake, ci 19140/yellow 5 lake, ci 42090/blue 1 lake, ci 75470/carmine]

Comparative Topcoat: trimethyl pentaphenyl trisiloxane, bis-diglyceryl polyacyladipate-2, ozokerite, cera alba/beeswax/cire dabeille, calcium sodium borosilicate, calcium aluminum borosilicate, tocopheryl acetate, fragrance. [+/- may contain mica, ci 77891/titanium dioxide, ci 77491/iron oxides]

### Example 5 -- Evaluation Methods And Test Results

Compositions were tested for tackiness, shine, and color transfer-resistance. Testing methods are described below.

### Tack Testing

The films of each formula were deposited onto contrast cards using a 3 MIL drawdown bar and an Automatic Drawdown Machine. The films were dried at room temperature (25 ° C) overnight and analyzed using a Texture Analyzer equipped with a ball probe. Tack force was measured after applying 350 g-force for 10 seconds. Then, the values of the tackiness were correlated to the comfort of wear of the tested products. The samples having the tackiness having values higher than 100 gr/force, were considered to be very uncomfortable to wear. The tack values between 50-100 gr/force, indicated medium comfort, and those with values of less than 50 gr/force were considered to be comfortable.

### Shine Testing

Films of each formula were deposited onto contrast cards using a 3 MIL drawdown bar and an Automatic Drawdown Machine. The films were dried at room temperature (25°C) overnight, then analyzed using a gloss meter (BYK: micro-TRI-gloss). For the system with top coats, the top coat was applied onto the color coat by hand to make sure film of top coat covering all area of color coats before measuring gloss.

### Color Transfer-Resistance Testing

The method described in the paragraph below is defined as "Method A" for purposes of this application.

Films of lipstick base coat (color coat) were deposited onto polyurethane white bioskin using a 3 MIL drawdown bar. Films were allowed to dry overnight. Each top coat was applied by hand with horizontal movements of the stick. Color transfer onto the stick was noted as yes (for when transfer occurred) and no (for when no transfer occurred). The top coat was allowed to stand onto the color coat for 5 minutes, then a KimWipe (folded 3 times) was put on top of the sample and the finger swiped across. Color transfer was quantified as 0 (no transfer), 1 (low transfer), 2 (medium transfer), 3 (high transfer). Top coat was reapplied in the same area to mimic real life top coat reapplication.

### Results

Table 1 below show results from testing where the long-wear lip composition in example 3 was used as the basecoat.

**Table 1**

| | Lip Composition Alone | Composition 1 | Invention Composition 2 | Comparative Commercial Topcoat |
|---|---|---|---|---|
| Tack | 28.4 | 15.4 | 19.3 | 14.8 |
| Shine (60°) | 3.3 | 47.4 | 49.3 | 49.1 |
| Color Transfer: First Application | NA | 1 | 1 | 2 |
| Color Transfer: Second Application | NA | 1 | 1 | 3 |

Table 2 below show results from testing where the long-wear lip composition in example 4 was used as the basecoat.

**Table 2**

| | Commercial Lip Composition Alone | Composition 1 | Invention Composition 2 | Comparative Commercial Topcoat |
|---|---|---|---|---|
| Tack | 92.6 | 5.7 | 5.0 | 9.3 |
| Shine (60°) | 8.5 | 33.7 | 35.9 | 55.7 |
| Color Transfer: First Application | NA | 1 | 1 | 1 |
| Color Transfer: Second Application | NA | 1 | 1 | 1 |

## Claims

1. A kit comprising (1) a long-wear lip composition comprising at least one silicone film-forming agent and at least one silicon elastomer, and (2) a topcoat composition comprising at least one phenylated silicone oil and at least one wax, wherein the topcoat composition does not comprise diglyceryl polyacyladipate in an amount which inhibits the transfer-resistance of the long-wear lip composition after application (as determined by "Method A" as defined in the description) wherein the topcoat composition comprises at least one silicone film-forming agent selected from the group consisting of silicone resins, polyorganosiloxane copolymers, and mixtures thereof.

2. The kit of claim 1, wherein the long-wear lip composition comprises:
from about 1% to about 30% by weight of at least one silicone elastomer;
from about 2% to about 30% by weight of at least one non-volatile oil;
from about 2% to about 35% by weight of at least one polyorganosiloxane copolymer;
from about 5% to about 30% by weight of at least one silicone resin;
from about 5% or about 50% of at least one volatile solvent;
optionally at least one wax;
optionally at least one colorant; and
optionally at least one filler;
wherein the ratio of the silicone elastomer to the at least one non-volatile oil is from about 1:0.02 to about 1:10; the weights being relative to the total weight of the long-wear lip composition, and
wherein the topcoat composition comprises trimethyl pentaphenyl trisiloxane and at least one wax selected from the group consisting of beeswax, paraffin wax, microcrystalline wax, synthetic wax, and mixtures thereof.

3. The kit of claim 1 or claim 2, wherein the topcoat composition does not comprise: (1) synthetic esters of formula R₁COOR₂ in which R₁ represents the residue of a linear or branched higher fatty acid containing from 7 to 40 carbon atoms and R₂ represents a branched hydrocarbon-based chain containing from 3 to 40 carbon atoms (2) C8-C26 higher fatty acids; or (3) C8-C26 higher fatty alcohols in an amount which inhibits the transfer-resistance of the long-wear lip composition after application. (as determined by "Method A" as defined in the description)

4. A method for making-up lips comprising applying a long-wear lip composition comprising at least one silicone film-forming agent and at least one silicon elastomer to the lips, and applying a topcoat composition comprising at least one phenylated silicone oil and at least one wax to the long-wear lip composition, wherein the topcoat composition does not comprise diglyceryl polyacyladipate in an amount which inhibits the transfer-resistance of the long-wear lip composition after application, (as determined by "Method A" as defined in the description), wherein the topcoat composition comprises at least one silicone film-forming agent selected from the group consisting of silicone resins, polyorganosiloxane copolymers, and mixtures thereof.

5. The method of claim 4, wherein when the topcoat composition is applied to the long-wear lip composition, the topcoat composition does not inhibit the transfer-resistance of the long-wear lip composition. (as determined by "Method A" as defined in the description)

6. A topcoat composition consisting of:
(a) about 75% to about 95% trimethyl pentaphenyl trisiloxane;
(b) about 1% to about 5% beeswax;
(c) about 5% to about 10% wax selected from the group consisting of ozokerite, paraffin wax, microcrystalline wax, synthetic wax, and mixtures thereof;
(d) 0% to about 5% silicone film forming agent;
(e) 0% to about 1% tocopheryl acetate;
(f) 0% to 10% sucrose acetate isobutyrate; and
(g) 0% to 0.25% fragrance;
said topcoat composition comprising a silicone film-forming agent selected from the group consisting of silicone resins, polyorganosiloxane copolymers, and mixtures thereof.

7. A topcoat composition according to claim 6 comprising tocopheryl acetate and sucrose acetate isobutyrate.

## Patentansprüche

1. Kit, umfassend (1) eine Long-Wear-Lippenzusammensetzung, umfassend wenigstens einen Silicon-Filmbildner und wenigstens ein Silizium-Elastomer, und (2) eine Topcoat-Zusammensetzung, umfassend wenigstens ein phenyliertes Siliconöl und wenigstens ein Wachs, wobei die Topcoat-Zusammensetzung kein Diglycerylpolyacyladipat in einer Menge umfasst, die die Übertragungsbeständigkeit der Long-Wear-Lippenzusammensetzung nach dem Auftragen (wie bestimmt durch das in der Beschreibung definierte "Verfahren A") beeinträchtigt, wobei die Topcoat-Zusammensetzung wenigstens einen Silicon-Filmbildner ausgewählt aus der Gruppe bestehend aus Siliconharzen, Polyorganosiloxan-Copolymeren und Gemischen davon umfasst.

2. Kit gemäß Anspruch 1, wobei die Long-Wear-Lippenzusammensetzung umfasst:
von etwa 1 Gew.-% bis etwa 30 Gew.-% an wenigstens einem Silicon-Elastomer;
von etwa 2 Gew.-% bis etwa 30 Gew.-% an wenigstens einem nichtflüchtigen Öl;
von etwa 2 Gew.-% bis etwa 35 Gew.-% an wenigstens einem Polyorganosiloxan-Copolymer;
von etwa 5 Gew.-% bis etwa 30 Gew.-% an wenigstens einem Siliconharz;
von etwa 5 Gew.-% bis etwa 50 Gew.-% an wenigstens einem flüchtigen Lösungsmittel;
gegebenenfalls wenigstens ein Wachs;
gegebenenfalls wenigstens einen Farbstoff; und
gegebenenfalls wenigstens einen Füllstoff;
wobei das Verhältnis des Silicon-Elastomers zu dem wenigstens einen nichtflüchtigen Öl von etwa 1:0,02 bis etwa 1:10 beträgt; wobei die Gewichte auf das Gesamtgewicht der Long-Wear-Lippenzusammensetzung bezogen sind, und
wobei die Topcoat-Zusammensetzung Trimethylpentaphenyltrisiloxan und wenigstens ein Wachs ausgewählt aus der Gruppe bestehend aus Bienenwachs, Paraffinwachs, mikrokristallinem Wachs, synthetischem Wachs und Gemischen davon umfasst.

3. Kit gemäß Anspruch 1 oder Anspruch 2, wobei die Topcoat-Zusammensetzung nicht umfasst: (1) synthetische Ester der Formel R₁COOR₂, wobei R₁ den Rest einer linearen oder verzweigten höheren Fettsäure, die von 7 bis 40 Kohlenstoffatome enthält, darstellt und R₂ eine verzweigte Kette auf Kohlenwasserstoffbasis, die von 3 bis 40 Kohlenstoffatome enthält, darstellt; (2) höhere C8-C26-Fettsäuren; oder (3) höhere C8-C26-Fettalkohole in einer Menge, die die Übertragungsbeständigkeit der Long-Wear-Lippenzusammensetzung nach dem Auftragen (wie bestimmt durch das in der Beschreibung definierte "Verfahren A") beeinträchtigt.

4. Verfahren zum Make-up von Lippen, umfassend Auftragen einer Long-Wear-Lippenzusammensetzung, die wenigstens einen Silicon-Filmbildner und wenigstens ein Silizium-Elastomer umfasst, auf die Lippen und Auftragen einer Topcoat-Zusammensetzung, die wenigstens ein phenyliertes Siliconöl und wenigstens ein Wachs umfasst, auf die Long-Wear-Lippenzusammensetzung, wobei die Topcoat-Zusammensetzung kein Diglycerylpolyacyladipat in einer Menge umfasst, die die Übertragungsbeständigkeit der Long-Wear-Lippenzusammensetzung nach dem Auftragen (wie bestimmt durch das in der Beschreibung definierte "Verfahren A") beeinträchtigt, wobei die Topcoat-Zusammensetzung wenigstens einen Silicon-Filmbildner ausgewählt aus der Gruppe bestehend aus Siliconharzen, Polyorganosiloxan-Copolymeren und Gemischen davon umfasst.

5. Verfahren gemäß Anspruch 4, wobei die Topcoat-Zusammensetzung auf die Long-Wear-Lippenzusammensetzung aufgetragen wird, wobei die Topcoat-Zusammensetzung die Übertragungsbeständigkeit der Long-Wear-Lippenzusammensetzung (wie bestimmt durch das in der Beschreibung definierte "Verfahren A") nicht beeinträchtigt.

6. Topcoat-Zusammensetzung, bestehend aus:
(a) etwa 75 % bis etwa 95 % Trimethylpentaphenyltrisiloxan;
(b) etwa 1 % bis etwa 5 % Bienenwachs;
(c) etwa 5 % bis etwa 10 % Wachs ausgewählt aus der Gruppe bestehend aus Ozokerit, Paraffinwachs, mikrokristallinem Wachs, synthetischem Wachs und Gemischen davon;
(d) 0 % bis etwa 5 % Silicon-Filmbildner;
(e) 0 % bis etwa 1 % Tocopherylacetat;
(f) 0 % bis 10 % Saccharoseacetatisobutyrat; und
(g) 0 % bis 0,25 % Duftstoff;
wobei die Topcoat-Zusammensetzung einen Silicon-Filmbildner ausgewählt aus der Gruppe bestehend aus Siliconharzen, Polyorganosiloxan-Copolymeren und Gemischen davon umfasst.

7. Topcoat-Zusammensetzung gemäß Anspruch 6, umfassend Tocopherylacetat und Saccharoseacetatisobutyrat.

## Revendications

1. Kit comprenant (1) une composition pour les lèvres longue tenue comprenant au moins un agent filmogène de silicone et au moins un élastomère de silicium, et (2) une composition de revêtement supérieur comprenant au moins une huile de silicone phénylée et au moins une cire, la composition de revêtement supérieur ne comprenant pas de polyacyladipate de diglycéryle en une quantité qui inhibe la résistance au transfert de la composition pour les lèvres longue tenue après l'application (comme déterminé par le « procédé A » comme défini dans la description)
la composition de revêtement supérieur comprenant au moins un agent filmogène de silicone choisi dans le groupe constitué par des résines de silicone, des copolymères de polyorganosiloxane, et des mélanges correspondants.

2. Kit selon la revendication 1, la composition pour les lèvres longue tenue comprenant :
d'environ 1 % à environ 30 % en poids d'au moins un élastomère de silicone ;
d'environ 2 % à environ 30 % en poids d'au moins une huile non volatile ;
d'environ 2 % à environ 35 % en poids d'au moins un copolymère de polyorganosiloxane ;
d'environ 5 % à environ 30 % en poids d'au moins une résine de silicone ;
d'environ 5 % ou environ 50 % d'au moins un solvant volatil ;
éventuellement au moins une cire ;
éventuellement au moins une matière colorante ; et
éventuellement au moins une charge ;
le rapport de l'élastomère de silicone sur l'au moins une huile non volatile étant d'environ 1 :
0,02 à environ 1 : 10 ; les poids étant par rapport au poids total de la composition pour les lèvres longue tenue, et
la composition de revêtement supérieur comprenant du triméthyl-pentaphényl-trisiloxane et au moins une cire choisie dans le groupe constitué par une cire d'abeilles, une cire de paraffine, une cire microcristalline, une cire synthétique, et des mélanges correspondants.

3. Kit selon la revendication 1 ou la revendication 2, la composition de revêtement supérieur ne comprenant pas : (1) d'esters synthétiques de formule R₁COOR₂ dans lesquels R₁ représente le radical d'un acide gras supérieur linéaire ou ramifié contenant de 7 à 40 atomes de carbone et R₂ représente une chaîne à base d'hydrocarbure ramifié contenant de 3 à 40 atomes de carbone (2) des acides gras supérieurs en C8-C26 ; ou (3) des alcools gras supérieurs en C8-C26 en une quantité qui inhibe la résistance au transfert de la composition pour les lèvres longue tenue après l'application (comme déterminé par le « procédé A » comme défini dans la description).

4. Procédé pour le maquillage des lèvres comprenant une application d'une composition pour les lèvres longue tenue comprenant au moins un agent filmogène de silicone et au moins un élastomère de silicium sur les lèvres, et une application d'une composition de revêtement supérieur comprenant au moins une huile de silicone phénylée et au moins une cire sur la composition pour les lèvres longue tenue, la composition de revêtement supérieur ne comprenant pas de polyacyladipate de diglycéryle en une quantité qui inhibe la résistance au transfert de la composition pour les lèvres longue tenue après l'application (comme déterminé par le « procédé A » comme défini dans la description),
la composition de revêtement supérieur comprenant au moins un agent filmogène de silicone choisi dans le groupe constitué par des résines de silicone, des copolymères de polyorganosiloxane, et des mélanges correspondants.

5. Procédé selon la revendication 4, dans laquelle lorsque la composition de revêtement supérieur est appliquée sur la composition pour les lèvres longue tenue, la composition de revêtement supérieur n'inhibe pas la résistance au transfert de la composition pour les lèvres longue tenue (comme déterminé par le « procédé A » comme défini dans la description).

6. Composition de revêtement supérieur constituée de :
(a) environ 75 % à environ 95 % de triméthyl-pentaphényl-trisiloxane ;
(b) environ 1 % à environ 5 % de cire d'abeilles ;
(c) environ 5 % à environ 10 % d'une cire choisie dans le groupe constitué par l'ozokérite, une cire de paraffine, une cire microcristalline, une cire synthétique, et des mélanges correspondants ;
(d) 0 % à environ 5 % d'un agent filmogène de silicone ;
(e) 0 % à environ 1 % d'acétate de tocophéryle ;
(f) 0 % à 10 % d'acétate isobutyrate de saccharose ; et
(g) 0 % à 0,25 % de parfum ;
ladite composition de revêtement supérieur comprenant un agent filmogène de silicone choisi dans le groupe constitué par des résines de silicone, des copolymères de polyorganosiloxane, et des mélanges correspondants.

7. Composition de revêtement supérieur selon la revendication 6 comprenant de l'acétate de tocophéryle et de l'acétate isobutyrate de saccharose.
